# EUROPÄISCHE PATENTSCHRIFT

(11) **EP 0 556 673 B1**
(45) Veröffentlichungstag und Bekanntmachung des Hinweises auf die Patenterteilung: **17.09.1997**
(21) Anmeldenummer: 93101841.0
(22) Anmeldetag: 05.02.1993
(51) Int. Cl.: C07C 317/42, C07D 295/26, C07C 311/47, C07D 295/155, A61K 31/155, A61K 31/18

(54) **Ortho-substituierte Benzoylguanidine, Verfahren zu ihrer Herstellung, ihre Verwendung als Medikament oder Diagnostikum sowie sie enthaltendes Medikament**
Ortho-substituted benzoylguanidines, process for their preparation, their use as medicament or diagnostic agent, as well as medicaments containing them
Benzoylguanidines ortho-substitués, procédé pour leur préparation, leur utilisation comme medicament ou agent diagnostique, ainsi que médicament les contenant

(30) Priorität: 15.02.1992 DE 4204576
(43) Veröffentlichungstag der Anmeldung: 25.08.1993
(73) Patentinhaber: HOECHST AKTIENGESELLSCHAFT, 65926 Frankfurt am Main (DE)
(72) Erfinder: Lang, Hans-Jochen, Dr., W-6238 Hofheim/Ts (DE); Weichert, Andreas, Dr., W-6000 Frankfurt/M (DE); Englert, Heinrich, Dr., W-6238 Hofheim/Ts (DE); Scholz, Wolfgang, Dr., W-6236 Eschborn (DE); Albus, Udo, Dr., W-6364 Florstadt (DE); Lang, Florian, Prof. Dr., W-7778 Markdorf (DE)

(56) Entgegenhaltungen:
- EP-A- 0 416 499
- US-A- 3 780 027

## Beschreibung

Die Erfindung betrifft ortho-substituierte Benzoylguanidine der Formel I 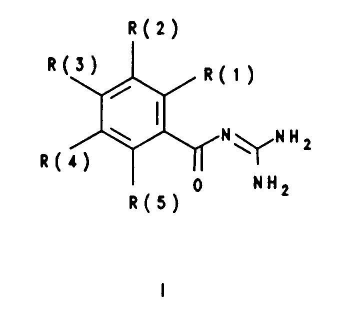 mit:
R(1) gleich F, Cl, Br, J, C₁-C₆-Alkyl, -X-R(6),
   X gleich O, S, NR(7) oder Y-ZO und
   Y gleich O oder NR(7) und
   Z gleich C oder SO,
   R(6) gleich H, C₁-C₆-Alkyl, C₅-C₇-Cycloalkyl, Cyclohexylmethyl, Cyclopentylmethyl, -(CH₂)ₘCₚF₂ₚ₊₁, -CₙH₂ₙ-R(8),
      m gleich Null oder 1,
      p gleich 1 - 3,
      n gleich Null bis 4,
      R(8) gleich Phenyl, das unsubstituiert ist oder substituiert mit 1-3 Substituenten aus den Gruppen F, Cl, CF₃, Methyl, Methoxy oder NR(9)R(10) mit R(9) und R(10) in der Bedeutung von H oder C₁-C₄-Alkyl,
   R(7) gleich H oder C₁-C₃-Alkyl,
      wobei R(6) und R(7) auch gemeinsam 4 oder 5 Methylengruppen sein können und eine CH₂-Gruppe durch ein O, S, NH, N-CH₃ oder N-Benzyl ersetzt sein kann,
R(3) gleich H, -X-R(6) mit
   X gleich O, S, NR(7) oder Y-ZO,
      R(7) gleich H oder C₁-C₃-Alkyl,
      Y gleich O oder NR(7),
      Z gleich C oder SO,
         wobei Y am Phenylrest der Formel I gebunden ist,
   R(6) gleich H, C₁-C₆-Alkyl, C₅-C₇-Cycloalkyl, Cyclohexylmethyl, Cyclopentylmethyl, -(CH₂)ₘCₚF₂ₚ₊₁, -CₙH₂ₙ-R(8),
      m gleich null oder 1,
      p gleich 1 - 3,
      n gleich null bis 4,
      R(8) gleich Phenyl, das unsubstituiert ist oder substituiert mit 1-3 Substituenten aus der Gruppe F, Cl, CF₃, Methyl, Methoxy, NR(9)R(10), R(9) und R(10) gleich H oder C₁-C₄-Alkyl,
   R(6) und R(7) auch gemeinsam 4 oder 5 Methylengruppen sein können und eine CH₂-Gruppe durch ein O, S, NH, N-CH₃ oder N-Benzyl ersetzt sein kann,
R(2) und R(4) - gleich oder verschieden - gleich R(11)-SO_{q}-, R(12)R(13)N-SO₂-, mit
   q gleich null - 2,
   R(11) gleich C₁-C₄-Alkyl, das unsubstituiert ist oder Phenyl als Substituenten trägt, wobei Phenyl unsubstituiert ist oder substituiert mit 1 - 3 Substituenten aus der Gruppe F, Cl, CF₃, Methyl, Methoxy oder NR(9)R(10) mit R(9) und R(10) gleich H oder C₁-C₄-Alkyl,
   R(12) und R(13) wie R(6) und R(7) definiert;
   oder einer der beiden Reste R(2) oder R(4) ist gleich Wasserstoff oder wie R(1) definiert,
R(5) H, Methyl, F, Cl, Methoxy,
sowie deren pharmazeutisch verträgliche Salze.

Bevorzugt sind Verbindungen der Formel I mit:
R(1) gleich F, Cl, C₁-C₃-Alkyl, CF₃, -X-R(6) mit
   X gleich O, S oder NR(7),
   R(6) gleich H, C₁-C₆-Alkyl, -(CH₂)ₘCₚF₂ₚ₊₁, -CₙH₂ₙ-R(8),
      m gleich null oder 1,
      p gleich 1 - 3,
      n gleich Null bis 4,
      R(8) gleich Phenyl, das unsubstituiert ist oder substituiert mit 1 - 3 Substituenten aus der Gruppe F, Cl, CF₃, Methyl, Methoxy, NR(9)R(10)
         mit R(9) und R(10) gleich H oder C₁-C₄-Alkyl,
      R(7) gleich H oder Methyl,
      wobei R(6) und R(7) auch gemeinsam 4 oder 5 Methylengruppen sein können und eine CH₂-Gruppe durch ein O, S, NH, N-CH₃ oder N-Benzyl ersetzt sein kann,
R(3) gleich H,
R(2) und R(4) - gleich oder verschieden - gleich CH₃-SO_{q}- oder R(12)R(13)N-SO₂-,
   q gleich Null - 2,
   R(12) und R(13) gleich R(6) und R(7),
   oder einer der beiden Reste R(2) oder R(4) ist gleich H oder wie R(1) definiert,
R(5) gleich H,
sowie deren pharmazeutisch verträgliche Salze.

Besonders bevorzugt sind Verbindungen der Formel I mit:
R(1) gleich F, Cl, C₁-C₃-Alkyl, Gruppe -X-R(6),
   X gleich O, S oder NR(7),
   R(6) gleich H, C₁-C₄-Alkyl, -CₙH₂ₙ-R(8),
      n gleich null bis 3,
      R(8) gleich Phenyl, das unsubstituiert ist oder substituiert mit 1-3 Substituenten aus der Gruppe F, Cl, CF₃, Methyl, Methoxy, NR(9)R(10) mit R(9) und R(10) gleich H oder Methyl,
      R(7) gleich H oder Methyl,
      wobei R(6) und R(7) auch gemeinsam 4 oder 5 Methylengruppen sein können und eine CH₂-Gruppe durch ein O, S, N-CH₃, N-Benzyl ersetzt sein kann,
R(2), R(3) und R(5) gleich Wasserstoff,
R(4) gleich CH₃-SO₂- oder R(12)R(13)N-SO₂- mit
   R(12) gleich -CₙH₂ₙ-R(8),
      n gleich 1 bis 3,
      R(8) gleich Phenyl, das unsubstituiert ist oder substituiert mit 1-3 Substituenten aus den Gruppen F, Cl, CF₃, Methyl, Methoxy oder NR(9)R(10) mit R(9) und R(10) in der Bedeutung von H oder Methyl,
   R(13) gleich H,
      oder R(12) und R(13) können auch gemeinsam 4 oder 5 Methylengruppen sein,
sowie deren pharmazeutisch verträgliche Salze.

Enthält einer der Substituenten R(1) bis R(13) ein Asymmetriezentrum, so gehören sowohl S als auch R konfigurierte Verbindungen zur Erfindung. Die Verbindungen können als optische Isomere, als Diastereomere, als Racemate oder als Gemische derselben vorliegen.

Die bezeichneten Alkylreste können sowohl geradkettig wie verzweigt vorliegen.

Die Erfindung betrifft weiterhin ein Verfahren zur Herstellung der Verbindungen I, dadurch gekennzeichnet, daß man Verbindungen der Formel II 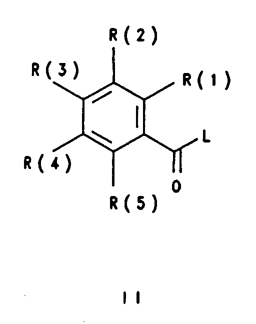 mit Guanidin umsetzt, worin R(1) bis R(5) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L=Cl), die man ihrerseits wiederum in an sich bekannter Weise aus den zugrundeliegenden Carbonsäuren (Formel II, L= OH) beispielsweise mit Thionylchlorid herstellen kann.

Neben den Carbonsäurechloriden der Formel II [L=Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L=OCH₃ durch Behandeln mit gasförmigem HCl in Methanol, die Imidazolide der Formel II durch Behandeln mit Carbonyldiimidazol (L= 1-Imidazolyl, Staab, Angew. Chem. Int. Ed. Engl. 1, 351-367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-6[(Cyano(ethoxycarbonyl)methylen)amino]-1,1,3,3-tetramethyluronium-tetrafluo borat ("TOTU") (siehe Weiss und Krommer, Chemiker Zeitung 98, 817 (1974)). Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren, aber inerten organischen Lösungsmittel. Dabei haben sich bei der Umsetzung der Benzoesäuremethylester (II, L=OMe) mit Guanidin Methanol oder THF zwischen 20°C und Siedetemperatur dieser Lösungsmittel bewährt. Bei den meisten Umsetzungen von Verbindungen II mit salzfreiem Guanidin III wurde vorteilhaft in aprotischen inerten Lösungsmitteln, wie THF, Dimethoxyethan, Dioxan, gearbeitet. Aber auch Wasser kann als Lösungsmittel bei der Umsetzung von II mit Guanidin verwendet werden.

Wenn L=Cl bedeutet, arbeitet man vorteilhaft unter Zusatz eines Säurefängers, z.B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden, indem man beispielsweise 2-Chlor- oder 2-Fluor-5-chlorsulfonyl-benzoesäure mit Ammoniak oder einem Amin in eine 5-Aminosulfonyl-2-chlor- oder -fluorbenzoesäure bzw. mit einem schwachen Reduktionsmittel wie Natriumbisulfit und anschließender Alkylierung in eine 5-Alkylsulfonyl-2-chlor- oder -2-fluorbenzoesäure überführt und die so erhaltenen Benzoesäure-Derivate nach einer der oben beschriebenen Verfahrensvarianten zu erfindungsgemäßen Verbindungen I umgesetzt werden.

Derartige Carbonsäuren oder deren Ester der Formel II mit L = -OH oder beispielsweise -O-Methyl und R(1) und/oder R(3) in der Bedeutung von Halogen können aber auch als Ausgangsverbindungen anderer Carbonsäuren dienen, wobei das Halogen in R(1)- und/oder R(4)-Position sehr bequem in bekannter Weise gegen zahlreiche nucleophile Reagenzien, wie Mercaptane R(6)-SH oder primäre Amine R(6)R(7)NH unter Bildung weiterer Benzoesäurederivate II mit L = -OH bzw. -O-Methyl ausgetauscht werden kann.

In ähnlicher Weise können ausgehend von 5-Nitro-2-chlorbenzoesäure durch nucleophile Einführung eines Restes R(1) in Position 2 (Austausch gegen Cl) und weiterer Abwandlung der Nitrogruppe, wie Reduktion zu NH₂ und nachfolgende Alkylierung oder Verdrängung, beispielsweise durch Diazotierung und Sandmeyer-Reaktion, weitere Benzoesäurederivate (II, L= -OH) hergestellt werden.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.

Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 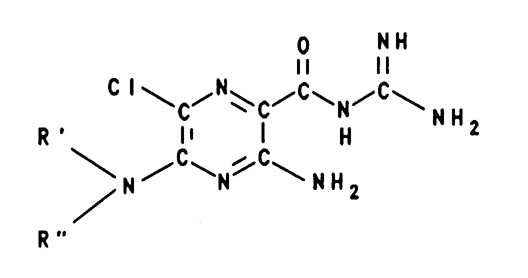
Amilorid: R',R''= H
Dimethylamilorid: R',R''= CH₃
Ethylisopropylamilorid: R'= C₂H₅, R''= CH(CH₃)₂

Darüber hinaus sind Untersuchungen bekannt geworden, die auf antiarrhythmische Eigenschaften von Amilorid hinweisen (Circulation 79, 1257-63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der europäischen Offenlegungsschrift 416 499 (HOE 89/F 288) werden Benzoylguanidine beschrieben, die in den den Resten R(1) und R(2) entsprechenden Stellungen Wasserstoff tragen. In der US-Patentschrift 3 780 027 werden Acylguanidine beschrieben, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet. Das in diesem Patent beschriebene N-Amidino-3-furfurylamino-4-Chlor-5-methylsulfonyl-benzamid wurde nachsynthetisiert und zeigte in unseren Modellen keine antiarrhythmische Wirkung.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge inhibition des zellulären Na+/H+ Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle inhibitoren des zellulären Natrium-Protonen-Antiporters (Na+/H+ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Losungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg bis 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 200 mg pro Tag notwendig werden.

### Experimenteller Teil

### Allgemeine Vorschrift zur Herstellung von Benzoyl-guanidinen (I) aus Benzoesäuren (II, L=OH)

Man löst bzw. suspendiert 0,01 M des Benzoesäurederivates der Formel II in 60 ml wasserfreiem Tetrahydrofuran (THF) und versetzt sodann mit 1,78 g (0,011 M) Carbonyldiimidazol. Nach dem Rühren über 2 Stunden bei Zimmertemperatur werden 2,95 g (0,05 M) Guanidin in die Reaktionslösung eingetragen. Nach dem Rühren über Nacht destilliert man das THF unter vermindertem Druck (Rotavapor) ab, versetzt mit Wasser, stellt mit 2N HCL auf pH 6-7 und filtriert das entsprechende Benzoylguanidin (Formel I) ab.
Die so erhaltenen Benzoylguanidine können durch Behandeln mit wäßriger oder methanolischer Salzsäure oder anderen pharmakologisch verträglichen Säuren in die entsprechenden Salze übergeführt werden.

### Beispiel 1:

2-Chlor-5-methylsulfonylbenzoyl-guanidin-hydrochlorid, 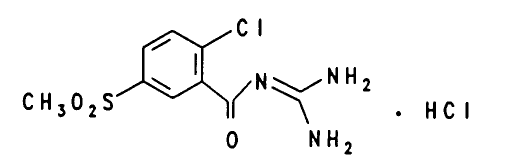 Farblose Kristalle, Schmp. 208-210°C.
aus 2-Chlor-5-methylsulfonylbenzoesäure (Schmp. 182-186°C),

### Beispiel 2:

2-Chlor-5-piperidylsulfonylbenzoyl-guanidin, 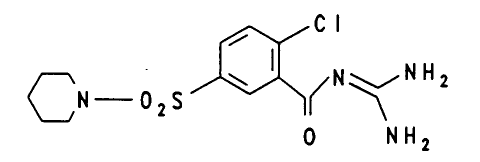 Farblose Kristalle, Schmp. 226-229°C.
aus 2-Chlor-5-piperidylsulfonylbenzoesäure (Schmp. 207-210°C),

### Beispiel 3:

2-Chlor-5-(1-pyrrolidinyl-sulfonyl)benzoyl-guanidin, 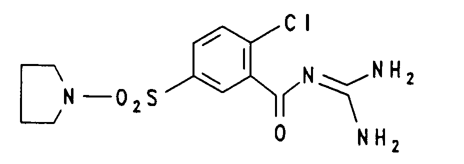 Farblose Kristalle, Schmp. 195-198°C.
aus 2-Chlor-5-(1-pyrrolidinyl-sulfonyl)benzoesäure (Schmp. 205°C),

### Beispiel 4:

2-Chlor-5-N-methylsulfamoylbenzoyl-guanidin-hydrochlorid, 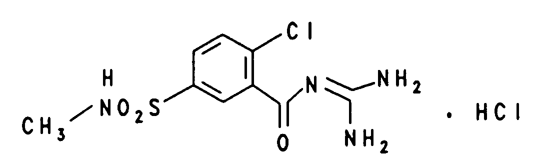 farblose Kristalle, Schmp. 201-203°C,
aus 2-Chlor-5-N-methylsulfamoylbenzoesäure (Schmp. 169-170°C),

### Beispiel 5:

2-Chlor-5-N,N-dipropylsulfamoylbenzoyl-guanidin-hydrochlorid, 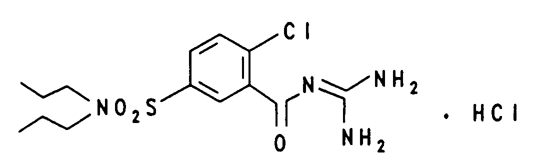 farblose Kristalle, Schmp. 170-173°C
aus 2-Chlor-5-N,N-dipropylsulfamoylbenzoesäure (Schmp. 102-104°C),

### Beispiel 6:

2-Chlor-5-(2-phenylethyl-sulfamoyl)benzoyl-guanidin-hydrochlorid, 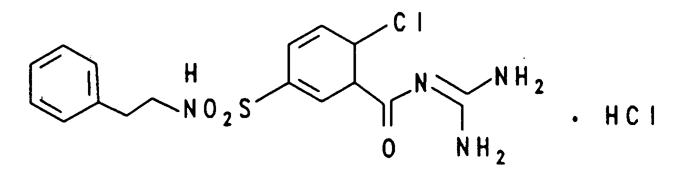 farblose Kristalle, Schmp. 70°C,
aus 2-Chlor-5-(2-phenylethyl-sulfamoyl)benzoesäure (Schmp. 160-163°C),

### Beispiel 7:

2-Chlor-5-N-methyl-N-(2-phenylethyl)sulfamoyl-benzoyl-guanidin-hydrochlorid, farblose Kristalle, Schmp. 186-188°C, 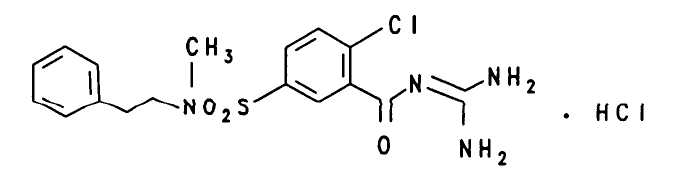 aus 2-Chlor-5-N-methyl-N-(2-phenylethyl)sulfamoyl-benzoesäure (Schmp.127-129°C).

### Beispiel 8:

2-Piperidyl-5-sulfamoylbenzoyl-guanidin, 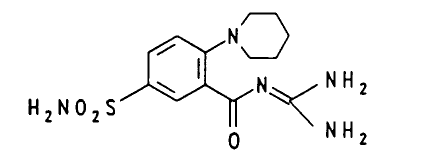 farblose Kristalle, Schmp. 247°C,
aus 2-Piperidyl-5-sulfamoylbenzoesäure (Schmp. 248°C, dargestellt aus 2-Chlor-5-sulfamoylbenzoesäure in 10 Mol Piperidin unter Inertgas für 24 Stunden unter Rückfluß kochen, abdestillieren des überschüssigen Piperidins und Behandeln des Rückstandes mit Wasser/verdünnter Salzsäure bei pH 1-2).

### Beispiel 9:

2-Benzylamino-5-sulfamoylbenzoyl-guanidin, 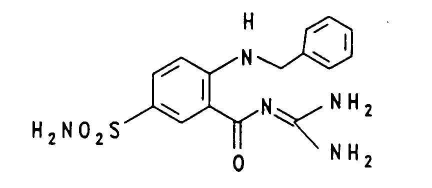 farblose Kristalle, Schmp. 191-193°C,
aus 2-Benzylamino-5-sulfamoylbenzoesäure (Schmp. 246°C, dargestellt aus 2-Chlor-5-sulfamoylbenzoesäure in 20 Äquivalenten Benzylamin über 8 Stunden bei 130°C/Inertgas und Behandeln mit Wasser/verdünnter HCl bei pH 1-2)

### Beispiel 10:

2-N-Methyl-N-(2-phenylethyl)amino-5-sulfamoylbenzoylguanidin-hydrochlorid, 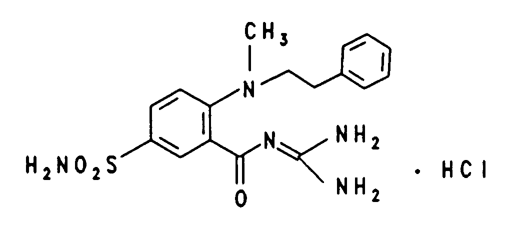 farblose Kristalle, Schmp. 180°C,
aus 2-N-Methyl-N-(2-phenylethyl)amino-5-sulfamoylbenzoesäure (Schmp. 240°C, Darstellung durch Erhitzen von 2-Fluor-5-sulfamoylbenzoesäure und N-Methyl-2-phenylethylamin über 10 -15 Stunden in Dimethylacetamid in Gegenwart von 4 Äquivalenten Ethyl-diisopropylamin auf 120°C, Verdampfen des Lösungsmittels und Behandeln des Rückstandes mit Wasser und verdünnter HCl bei pH 1-2),

### Beispiel 11:

2-N-Methyl-N-(2-phenylethyl)amino-5-methylsulfonylbenzoyl-guanidin-hydrochlorid, 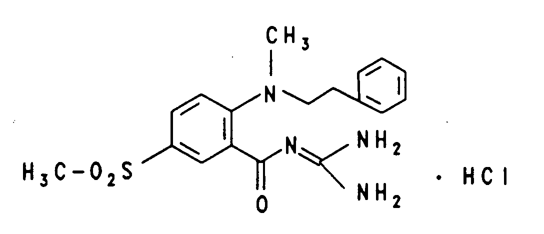 farblose Kristalle, Schmp. 123°C,
aus 2-N-Methyl-N-(2-phenylethyl)amino-5-methylsulfonylbenzoesäure (amorphes Öl, Darstellung aus 2-Chlor-5-methylsulfonylbenzoesäure und N-Methyl-2-phenylethylamin analog Beispiel 10 über 8 Stunden bei 140°C).

### Beispiel 12:

5-N-Methyl-N-(2-phenylethyl)sulfamoyl-2-piperidinobenzoyl-guanidin, 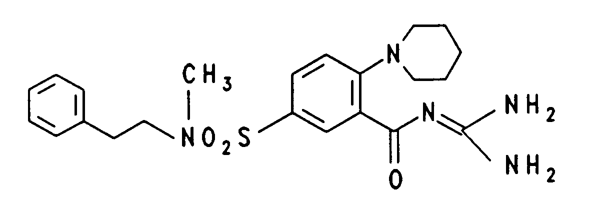 farblose Kristalle, Schmp. 85°C,
aus 5-N-Methyl-N-(2-phenylethyl)sulfamoyl-2-piperidinobenzoesäure (amorphes Zwischenprodukt ohne definierten Schmp., Darstellung aus 2-Chlor-5-N-Methyl-N-(2-phenylethyl)sulfamoylbenzoesäure und Piperidin analog Beispiel 8),

### Beispiel 13:

2-(2-Chlorphenylthio)-5-methylsulfonylbenzoyl-guanidin-hydrochlorid, 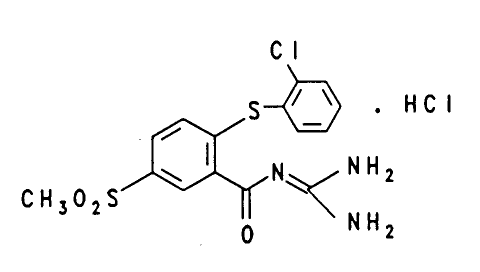 farblose Kristalle, Schmp. 284-286°C
aus 2-(2-Chlorphenylthio)-5-methylsulfonylbenzoesäure (Schmp. 210-216°C, dargestellt durch Umsetzung von 2-Chlor-5-methylsulfonyl-benzoesäuremethylester mit 1 Äqu. 2-Chlorthiophenol und überschüssigem K₂CO₃ in DMF bei 90°C über 7 Stunden und Hydrolyse des so erhaltenen 2-(2-Chlorphenylthio)-5-methylsulfonyl-benzoesäuremethylesters (Schmp. 145°C) in einem Gemisch aus Dioxan und Natronlauge bei Zimmertemperatur).

### Beispiel 14:

2-(2,6-Dichlorphenylthio)-5-methylsulfonylbenzoyl-guanidin-hydrochlorid, 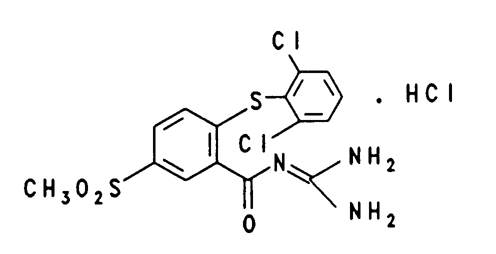 farblose Kristalle, Schmp. 288-290°C,
aus 2-(2,6-Dichlorphenylthio)-5-methylsulfonylbenzoesäure (Schmp. 244°C, dargestellt analog Beispiel 13 aus 2-Chlor-5-methylsulfonyl-benzoesäuremethylester mit 1 Äqu. 2,6-Dichlorthiophenol und Hydrolyse des so erhaltenen 2-(2,6-Dichlorphenylthio)-5-methylsulfonyl-benzoesäuremethylesters (Schmp. 139°C) analog Beispiel 13.

### Beispiel 15:

5-Methylsulfonyl-2-piperidinobenzoyl-guanidin-hydrochlorid, 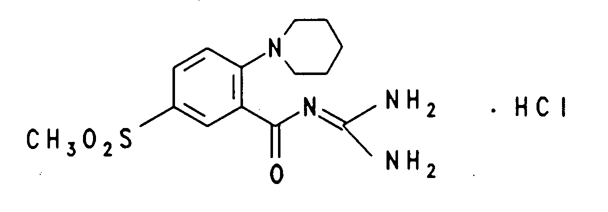 farblose Kristalle, Schmp. 124-130°C,
dargestellt aus 5-Methylsulfonyl-2-piperidinobenzoesäure (Schmp. 198°C, Darstellung durch Umsetzung von 2-Chlor-5-methylsulfonylbenzoesäure durch mehrstündiges Kochen in 10 Äquivalenten Piperidin am Rückflußkühler, Verdampfen des überschüssigen Piperidins und nachfolgendes Behandeln mit Wasser/verdünnter HCl bei pH 1-2),

### Beispiel 16

5-Methylsulfamoyl-2-piperidylbenzoyl-guanidin-hydrochlorid 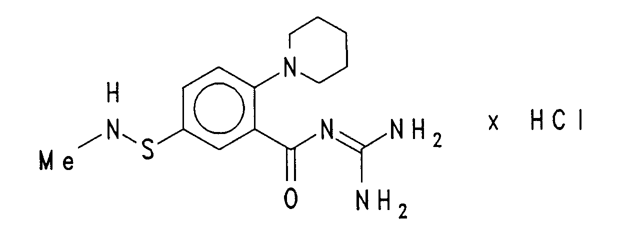 farblose Kristalle, Schmp. 212-214°C,
durch Umsetzung von 5-Methylsulfamoyl-2-piperidylbenzoesäure mit Guanidin gemäß der oben beschriebenen allgemeinen Vorschrift.

### Beispiel 17

2,3-Dichlor-5-methylsulfonylbenzoyl-guanidin-hydrochlorid, 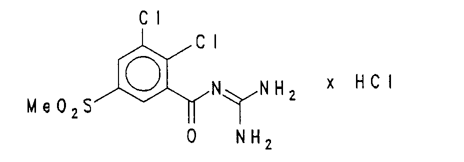 farblose Kristalle, Schmp. 280°C,
durch Umsetzung von 2,3-Dichlor-5-methylsulfonylbenzoesäure mit Guanidin gemäß der oben beschriebenen allgemeinen Vorschrift.

### Beispiel 18

2-Methyl-5-methylsulfonylbenzoyl-guanidin-hydrochlorid 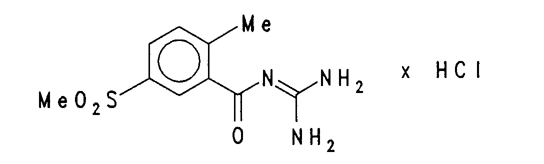 farblose Kristalle, Schmp. 212°C,
durch Umsetzung von 2-Methyl-5-methylsulfonylbenzoesäure mit Guanidin gemäß der oben beschriebenen allgemeinen Vorschrift.

### Beispiel 19

2-(2-Chlorbenzylamino)-5-sulfamoylbenzoyl-guanidin-hydrochlorid 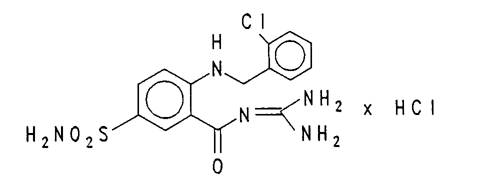 farblose Kristalle, Schmp. 137°C,
durch Umsetzung von 2-(2-Chlorbenzylamino)-5-sulfamoylbenzoesäure mit Guanidin gemäß der oben beschriebenen allgemeinen Vorschrift.

## Patentansprüche

1. Ortho-substituierte Benzoylguanidine der Formel I 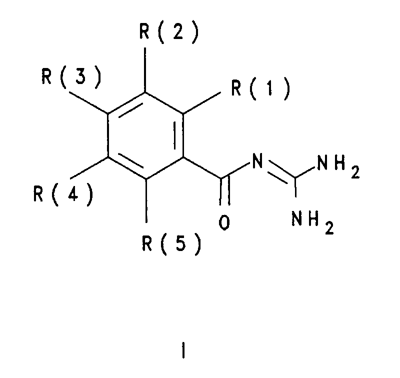 dadurch gekennzeichnet, daß die Substituenten wie folgt definiert sind:
R(1) gleich F, Cl, Br, J, C₁-C₆-Alkyl, -X-R(6),
X gleich O, S, NR(7) oder Y-ZO und
Y gleich O oder NR(7) und
Z gleich C oder SO,
R(6) gleich H, C₁-C₆-Alkyl, C₅-C₇-Cycloalkyl, Cyclohexylmethyl, Cyclopentylmethyl, -(CH₂)ₘCₚF₂ₚ₊₁, -CₙH₂ₙ-R(8),
m gleich Null oder 1,
p gleich 1 - 3,
n gleich Null bis 4,
R(8) gleich Phenyl, das unsubstituiert ist oder substituiert mit 1-3 Substituenten aus den Gruppen F, Cl, CF₃, Methyl, Methoxy oder NR(9)R(10) mit R(9) und R(10) in der Bedeutung von H oder C₁-C₄-Alkyl,
R(7) gleich H oder C₁-C₃-Alkyl,
wobei R(6) und R(7) auch gemeinsam 4 oder 5 Methylengruppen sein können und eine CH₂-Gruppe durch ein O, S, NH, N-CH₃ oder N-Benzyl ersetzt sein kann,
R(3) gleich H, -X-R(6) mit
X gleich O, S, NR(7) oder Y-ZO,
R(7) gleich H oder C₁-C₃-Alkyl,
Y gleich O oder NR(7),
Z gleich C oder SO,
wobei Y am Phenylrest der Formel I gebunden ist,
R(6) gleich H, C₁-C₆-Alkyl, C₅-C₇-Cycloalkyl, Cyclohexylmethyl, Cyclopentylmethyl, -(CH₂)ₘCₚF₂ₚ₊₁, -CₙH₂ₙ-R(8),
m gleich null oder 1,
p gleich 1 - 3,
n gleich null bis 4,
R(8) gleich Phenyl, das unsubstituiert ist oder substituiert mit 1-3 Substituenten aus der Gruppe F, Cl, CF₃, Methyl, Methoxy, NR(9)R(10),
R(9) und R(10) gleich H oder C₁-C₄-Alkyl,
R(6) und R(7) auch gemeinsam 4 oder 5 Methylengruppen sein können und eine CH₂-Gruppe durch ein O, S, NH, N-CH₃ oder N-Benzyl ersetzt sein kann,
R(2) und R(4) - gleich oder verschieden - gleich R(11)-SO_{q}-, R(12)R(13)N-SO₂-, mit
q gleich null - 2,
R(11) gleich C₁-C₄-Alkyl, das unsubstituiert ist oder Phenyl als Substituenten trägt, wobei Phenyl unsubstituiert ist oder substituiert mit 1 - 3 Substituenten aus der Gruppe F, Cl, CF₃, Methyl, Methoxy oder NR(9)R(10) mit R(9) und R(10) gleich H oder C₁-C₄-Alkyl,
R(12) und R(13) wie R(6) und R(7) definiert;
oder einer der beiden Reste R(2) oder R(4) ist gleich Wasserstoff oder wie R(1) definiert,
R(5) H, Methyl, F, Cl, Methoxy,
sowie deren pharmazeutisch verträgliche Salze.

2. Verbindung nach Anspruch 1, dadurch gekennzeichnet, daß die Substituenten wie folgt definiert sind:
R(1) gleich F, Cl, C₁-C₃-Alkyl, CF₃, -X-R(6) mit
X gleich O, S oder NR(7),
R(6) gleich H, C₁-C₆-Alkyl, -(CH₂)ₘCₚF₂ₚ₊₁, -CₙH₂ₙ-R(8),
m gleich null oder 1,
p gleich 1 - 3,
n gleich Null bis 4,
R(8) gleich Phenyl, das unsubstituiert ist oder substituiert mit 1 - 3 Substituenten aus der Gruppe F, Cl, CF₃, Methyl, Methoxy, NR(9)R(10)
mit R(9) und R(10) gleich H oder C₁-C₄-Alkyl,
R(7) gleich H oder Methyl,
wobei R(6) und R(7) auch gemeinsam 4 oder 5 Methylengruppen sein können und eine CH₂-Gruppe durch ein O, S, NH, N-CH₃ oder N-Benzyl ersetzt sein kann,
R(3) gleich H,
R(2) und R(4) - gleich oder verschieden - gleich CH₃-SO_{q}- oder R(12)R(13)N-SO₂-,
q gleich Null - 2,
R(12) und R(13) gleich R(6) und R(7),
oder einer der beiden Reste R(2) oder R(4) ist gleich H oder wie R(1) definiert,
R(5) gleich H.

3. Verbindung I nach Anspruch 1, dadurch gekennzeichnet, daß die Substituenten wie folgt definiert sind:
R(1) gleich F, Cl, C₁-C₃-Alkyl, Gruppe -X-R(6),
X gleich O, S oder NR(7),
R(6) gleich H, C₁-C₄-Alkyl, -CₙH₂ₙ-R(8),
n gleich null bis 3,
R(8) gleich Phenyl, das unsubstituiert ist oder substituiert mit 1-3 Substituenten aus der Gruppe F, Cl, CF₃, Methyl, Methoxy, NR(9)R(10) mit R(9) und R(10) gleich H oder Methyl,
R(7) gleich H oder Methyl,
wobei R(6) und R(7) auch gemeinsam 4 oder 5 Methylengruppen sein können und eine CH₂-Gruppe durch ein O, S, N-CH₃ oder N-Benzyl ersetzt sein kann,
R(2), R(3) und R(5) gleich Wasserstoff,
R(4) gleich CH₃SO₂- oder R(12)R(13)N-SO₂- mit
R(12) gleich -CₙH₂ₙ-R(8),
n gleich 1 bis 3,
R(8) gleich Phenyl, das unsubstituiert ist oder substituiert mit 1-3 Substituenten aus den Gruppen F, Cl, CF₃, Methyl, Methoxy oder NR(9)R(10) mit R(9) und R(10) in der Bedeutung von H oder Methyl,
R(13) gleich H,
oder R(12) und R(13) können auch gemeinsam 4 oder 5 Methylengruppen sein.

4. Verfahren zum Herstellen einer Verbindung I nach Anspruch 1, dadurch gekennzeichnet, daß man
Verbindungen der Formel II 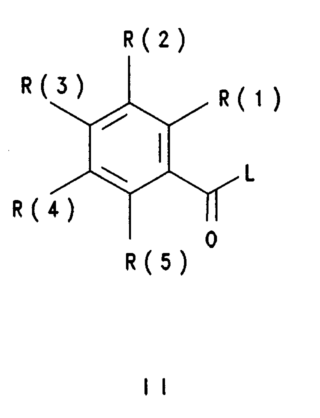 mit Guanidin umsetzt, worin R(1) bis R(5) die angegebene Bedeutung besitzen und L für eine leicht nucleophil substituierbare leaving group steht.

5. Verwendung einer Verbindung I nach Anspruch 1 zum Herstellen eines Medikaments zur Behandlung und zur Prophylaxe von Herz-Rhytmus-Störungen und von ischämisch induzierten Schäden.

6. Verwendung einer Verbindung I nach Anspruch 1 zum Herstellen eines Medikaments zur Inhibition der Proliferation von Zellen.

7. Medikament zum Behandeln und zur Prophylaxe von Herz-Rhytmus-Störungen und von ischämisch induzierten Schäden, dadurch gekennzeichnet, daß es eine wirksame Menge einer Verbindung I nach Anspruch 1 und pharmazeutisch übliche Zuschlagstoffe enthält.

8. Medikament zur Inhibition der Proliferation von Zellen, dadurch gekennzeichnet, daß es eine wirksame Menge einer Verbindung I nach Anspruch 1 und pharmazeutisch übliche Zuschlagstoffe enthält.

## Claims

1. An ortho-substituted benzoylguanidine of the formula I 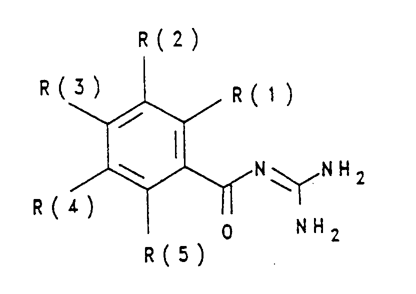 wherein the substituents are defined as follows:
R(1) is F, Cl, Br, I, C₁-C₆-alkyl or -X-R(6),
X is O, S, NR(7) or Y-ZO and
Y is O or NR(7) and
Z is C or SO,
R(6) is H, C₁-C₆-alkyl, c₅-C₇-cycloalkyl, cyclohexylmethyl, cyclopentylmethyl, -(CH₂)ₘCₚF₂ₚ₊₁ or -CₙH₂ₙ-R(8),
m is zero or 1,
p is 1 - 3,
n is zero to 4,
R(8) is phenyl which is unsubstituted or substituted by 1-3 substituents from the groups F, Cl, CF₃, methyl, methoxy or NR(9)R(10) where R(9) and R(10) have the meaning of H or C₁-C₄-alkyl,
R(7) is H or C₁-C₃-alkyl,
where R(6) and R(7) can also together be 4 or 5 methylene groups and a CH₂ group can be replaced by O, S, NH, N-CH₃ or N-benzyl,
R(3) is H or -X-R(6) where
X is O, S, NR(7) or Y-ZO,
R(7) is H or C₁-C₃-alkyl,
Y is O or NR(7),
Z is C or SO,
where Y is bonded to the phenyl radical in the formula I,
R(6) is H, C₁-C₆-alkyl, C₅-C₇-cycloalkyl, cyclohexylmethyl, cyclopentylmethyl, -(CH₂)ₘCₚF₂ₚ₊₁ or -CₙH₂ₙ-R(8),
m is zero or 1,
p is 1 - 3,
n is zero to 4,
R(8) is phenyl which is unsubstituted or substituted by 1-3 substituents from the group comprising F, Cl, CF₃, methyl, methoxy and NR(9)R(10),
R(9) and R(10) are H or C₁-C₄-alkyl,
R(6) and R(7) can also together be 4 or 5 methylene groups and a CH₂ group can be replaced by O, S, NH, N-CH₃ or N-benzyl,
R(2) and R(4) - identical or different - are R(11)-SO_{q}- or R(12)R(13)N-SO₂-, where
q is zero - 2,
R(11) is C₁-C₄-alkyl which is unsubstituted or carries phenyl as a substituent, where phenyl is unsubstituted or substituted by 1 - 3 substituents from the group comprising F, Cl, CF₃, methyl, methoxy and NR(9)R(10) where R(9) and R(10) are H or C₁-C₄-alkyl,
R(12) and R(13) are defined as R(6) and R(7); or one of the two radicals R(2) or R(4) is hydrogen or is defined as R(1),
R(5) is H, methyl, F, Cl or methoxy,
and their pharmaceutically tolerable salts.

2. A compound as claimed in claim 1, wherein the substituents are defined as follows:
R(1) is F, Cl, C₁-C₃-alkyl, CF₃ or -X-R(6) where
X is O, S or NR(7),
R(6) is H, C₁-C₆-alkyl, -(CH₂)ₘCₚF₂ₚ₊₁ or -CₙH₂ₙ-R(8),
m is zero or 1,
p is 1 - 3,
n is zero to 4,
R(8) is phenyl which is unsubstituted or substituted by 1 - 3 substituents from the group comprising F, Cl, CF₃, methyl, methoxy and NR(9)R(10)
where R(9) and R(10) are H or C₁-C₄-alkyl,
R(7) is H or methyl,
where R(6) and R(7) can also together be 4 or 5 methylene groups and a CH₂ group can be replaced by O, S, NH, N-CH₃ or N-benzyl,
R(3) is H,
R(2) and R(4) - identical or different - are CH₃-SO_{q}-or R(12)R(13)N-SO₂-,
q is zero - 2,
R(12) and R(13) are R(6) and R(7),
or one of the two radicals R(2) or R(4) is H or is defined as R(1),
R(5) is H.

3. A compound I as claimed in claim 1, wherein the substituents are defined as follows:
R(1) is F, Cl, C₁-C₃-alkyl or a group -X-R(6),
X is O, S or NR(7),
R(6) is H, C₁-C₄-alkyl or -CₙH₂ₙ-R(8),
n is zero to 3,
R(8) is phenyl which is unsubstituted or substituted by 1-3 substituents from the group comprising F, Cl, CF₃, methyl, methoxy and NR(9)R(10) where R(9) and R(10) are H or methyl,
R(7) is H or methyl,
where R(6) and R(7) can also together be 4 or 5 methylene groups and a CH₂ group can be replaced by O, S, N-CH₃ or N-benzyl,
R(2), R(3) and R(5) are hydrogen,
R(4) is CH₃SO₂- or R(12)R(13)N-SO₂- where
R(12) is -CₙH₂ₙ-R(8),
n is 1 to 3,
R(8) is phenyl which is unsubstituted or substituted by 1-3 substituents from the groups F, Cl, CF₃, methyl, methoxy or NR(9)R(10) where R(9) and R(10) have the meaning of H or methyl,
R(13) is H,
or R(12) and R(13) can also together be 4 or 5 methylene groups.

4. A process for the preparation of a compound I as claimed in claim 1, which comprises
reacting a compound of the formula II 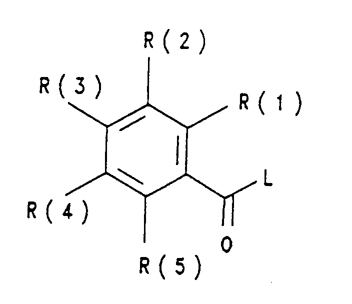 with guanidine, in which R(1) to R(5) have the given meaning and L is a leaving group which can be easily nucleophilically substituted.

5. The use of a compound I as claimed in claim 1 for preparing a medicament for the treatment and for the prophylaxis of cardiac arrhythmias and of ischemically induced damage.

6. The use of a compound I as claimed in claim 1 for preparing a medicament for inhibiting the proliferation of cells.

7. A medicament for the treatment and for the prophylaxis of cardiac arrhythmias and of ischemically induced damage, which contains an effective amount of a compound I as claimed in claim 1 and pharmaceutically customary additives.

8. A medicament for inhibiting the proliferation of cells, which contains an effective amount of a compound I as claimed in claim 1 and pharmaceutically customary additives.

## Revendications

1. Benzoylguanidines substituées en position ortho, de formule I 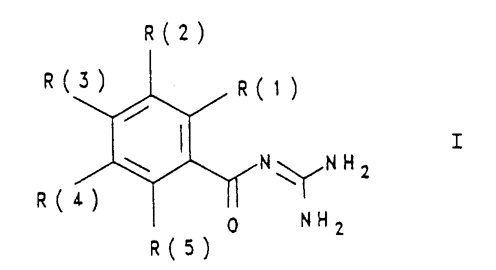 caractérisées en ce que les substituants sont définis comme suit:
R(1) représente F, Cl, Br, I ou un groupe alkyle en C₁-C₆ ou -X-R(6),
X représentant O, S ou un groupe NR(7) ou Y-ZO et
Y représentant O ou NR(7) et
Z représentant C ou SO,
R(6) représentant H ou un groupe alkyle en C₁-C₆, cycloalkyle en C₅-C₇, cyclohexylméthyle, cyclopentylméthyle, -(CH₂)ₘCₚF₂ₚ₊₁, -CₙH₂ₙ-R(8),
m étant égal à zéro ou 1,
p allant de 1 à 3,
n allant de zéro à 4,
R(8) représentant un groupe phényle qui est non substitué ou substitué par 1-3 substituants choisis parmi
F, Cl, CF₃, un groupe méthyle, méthoxy ou NR(9)R(10), R(9) et R(10) représentant H ou un groupe alkyle en C₁-C₄,
R(7) représentant H ou un groupe alkyle en C₁-C₃,
R(6) et R(7) pouvant également être ensemble 4 ou 5 groupes méthylène et un groupe CH₂ pouvant être remplacé par O, S ou par un groupe NH, N-CH₃ ou N-benzyle,
R(3) représente H ou un groupe -X-R(6),
X représentant O, S ou un groupe NR(7) ou Y-ZO,
R(7) représentant H ou un groupe alkyle en C₁-C₃,
Y représentant O ou NR(7),
Z représentant C ou SO,
Y étant lié au radical phényle de la formule I,
R(6) représentant H ou un groupe alkyle en C₁-C₆, cycloalkyle en C₅-C₇, cyclohexylméthyle, cyclopentylméthyle, -(CH₂)ₘCₚF₂ₚ₊₁, -CₙH₂ₙ-R(8),
m étant égal à zéro ou 1,
p allant de 1 à 3,
n allant de zéro à 4,
R(8) représentant un groupe phényle qui est non substitué ou substitué par 1-3 substituants choisis parmi
F, Cl, CF₃, un groupe méthyle, méthoxy ou NR(9)R(10), R(9) et R(10) représentant H ou un groupe alkyle en C₁-C₄,
R(6) et R(7) pouvant également être ensemble 4 ou 5 groupes méthylène et un groupe CH₂ pouvant être remplacé par O, S ou par un groupe NH, N-CH₃ ou N-benzyle,
R(2) et R(4) - identiques ou différents - représentent un groupe R(11)-SO_{q}-, R(12)R(13)N-SO₂-,
q allant de 0 à 2,
R(11) représentant un groupe alkyle en C₁-C₄ qui est non substitué ou porte un groupe phényle en tant que substituant, le groupe phényle étant non substitué ou portant 1-3 substituants choisis parmi F, Cl, CF₃, un groupe méthyle, méthoxy ou NR(9)R(10), R(9) et R(10) représentant H ou un groupe alkyle en C₁-C₄,
R(12) et R(13) étant définis comme R(6) et R(7); ou l'un des deux radicaux R(2) et R(4) représente un atome d'hydrogène ou est défini comme R(1),
R(5) représente H, F, Cl ou le groupe méthyle ou méthoxy, ainsi que leurs sels pharmaceutiquement acceptables.

2. Composé selon la revendication 1, caractérisé en ce que les substituants sont définis comme suit:
R(1) représente F, Cl ou un groupe alkyle en C₁-C₃ ou -X-R(6),
X représentant O, S ou un groupe NR(7),
R(6) représentant H ou un groupe alkyle en C₁-C₆, -(CH₂)ₘCₚF₂ₚ₊₁, -CₙH₂ₙ-R(8),
m étant égal à zéro ou 1,
p allant de 1 à 3,
n allant de zéro à 4,
R(8) représentant un groupe phényle qui est non substitué ou substitué par 1-3 substituants choisis parmi
F, Cl, CF₃, un groupe méthyle, méthoxy ou NR(9)R(10),
R(9) et R(10) représentant H ou un groupe alkyle en C₁-C₄,
R(7) représentant H ou le groupe méthyle,
R(6) et R(7) pouvant également être ensemble 4 ou 5 groupes méthylène et un groupe CH₂ pouvant être remplacé par O, S ou par un groupe NH, N-CH₃ ou N-benzyle,
R(3) représente H,
R(2) et R(4) - identiques ou différents - représentent un groupe CH₃-SO_{q}- ou R(12)R(13)N-SO₂-,
q allant de 0 à 2,
R(12) et R(13) étant définis comme R(6) et R(7); ou l'un des deux radicaux R(2) et R(4) représente un atome d'hydrogène ou est défini comme R(1),
R(5) représente H.

3. Composé I selon la revendication 1, caractérisé en ce que les substituants sont définis comme suit:
R(1) représente F, Cl ou un groupe alkyle en C₁-C₃ ou -X-R(6),
X représentant O, S ou un groupe NR(7),
R(6) représentant H ou un groupe alkyle en C₁-C₆, -CₙH₂ₙ-R(8),
n allant de 0 à 3,
R(8) représentant un groupe phényle qui est non substitué ou substitué par 1-3 substituants choisis parmi
F, Cl, CF₃, un groupe méthyle, méthoxy ou NR(9)R(10),
R(9) et R(10) représentant H ou le groupe méthyle,
R(7) représentant H ou le groupe méthyle,
R(6) et R(7) pouvant également être ensemble 4 ou 5 groupes méthylène et un groupe CH₂ pouvant être remplacé par O, S ou par un groupe N-CH₃ ou N-benzyle,
R(2), R(3) et R(5) représentent des atomes d'hydrogène,
R(4) représente un groupe CH₃-SO₂- ou R(12)R(13)N-SO₂-,
R(12) représentant un groupe -CₙH₂ₙ-R(8),
n allant de 0 à 3,
R(8) représentant un groupe phényle qui est non substitué ou substitué par 1-3 substituants choisis parmi
F, Cl, CF₃, un groupe méthyle, méthoxy ou NR(9)R(10),
R(9) et R(10) représentant H ou le groupe méthyle,
R(13) représentant H,
ou R(12) et R(13) pouvant également être ensemble 4 ou 5 groupes méthylène.

4. Procédé pour la préparation d'un composé I selon la revendication 1, caractérisé en ce que l'on fait réagir avec de la guanidine des composés de formule II 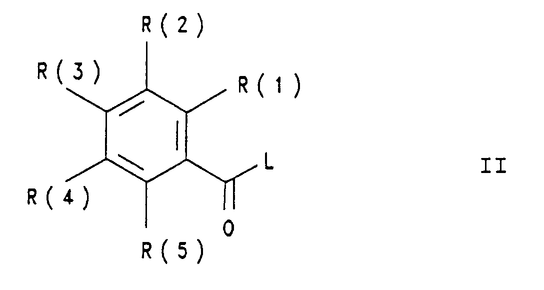 dans laquelle R(1) à R(5) ont les significations indiquées et L représente un groupe partant aisément remplaçable par substitution nucléophile.

5. Utilisation d'un composé I selon la revendication 1, pour la fabrication d'un médicament destiné au traitement et à la prophylaxie de troubles du rythme cardiaque et de dommages d'origine ischémique.

6. Utilisation d'un composé I selon la revendication 1, pour la fabrication d'un médicament destiné à l'inhibition de la prolifération de cellules.

7. Médicament destiné au traitement et à la prophylaxie de troubles du rythme cardiaque et de dommages d'origine ischémique, caractérisé en ce qu'il contient une quantité efficace d'un composé I selon la revendication 1 et des additifs utilisés habituellement en pharmacie.

8. Médicament destiné à l'inhibition de la prolifération de cellules, caractérisé en ce qu'il contient une quantité efficace d'un composé I selon la revendication 1 et des additifs utilisés habituellement en pharmacie.
